# EUROPEAN PATENT APPLICATION

(11) **EP 2 995 345 A1**
(43) Date of publication of application: **16.03.2016**
(21) Application number: 15184439.6
(22) Date of filing: 09.09.2015
(51) Int. Cl.: A61N 1/39, A61B 5/046

(54) **WEARABLE CARDIAC DEFIBRILLATOR SYSTEM DIAGNOSING DIFFERENTLY DEPENDING ON MOTION**

(30) Priority: 12.09.2014 US 201414485594
(71) Applicant: West Affum Holdings Corp., 1-1104 Grand Cayman (KY)
(72) Inventor: Sullivan, Joseph L., Kirkland, Washington 98034 (US); Marx, Robert P., Kent, Washington 98031 (US); Lu, Zhong Qun, Everett, Washington 98208 (US)
(74) Representative: Prinz & Partner mbB

(57) **Abstract**

Embodiments of a Wearable Cardiac Defibrillator (WCD) system include a measurement circuit that can render a physiological input from the patient (82). Such WCD systems may also receive a motion detection input that reveals whether a motion event has been detected by a motion detector. Optionally, a value becomes assigned to a motion level parameter in response to whether a motion event was detected or not, and the rhythm analysis can be based on the physiological input and on the assigned value. Optionally, a rhythm analysis of the physiological input may be performed in different manners, depending on whether or not a motion event has been detected. Optionally, a different shock/no shock criterion may be applied to the rhythm analysis, depending on whether or not a motion event has been detected. The patient (82) may receive an electrical shock (111) according to a shock/no shock determination.

## Description

### CROSS REFERENCE TO RELATED PATENT APPLICATIONS

This patent application claims priority from US Patent Application Serial No. 14/485,594, filed on September 12, 2014, all commonly assigned herewith, the disclosure of which is hereby incorporated by reference for all purposes.

### BACKGROUND

When people suffer from some types of heart arrhythmias, the result may be that blood flow to various parts of the body is reduced. Some arrhythmias may even result in a Sudden Cardiac Arrest (SCA). SCA can lead to death very quickly, e.g. within 10 minutes, unless treated in the interim.

Some people have an increased risk of SCA. People at a higher risk include individuals who have had a heart attack, or a prior SCA episode. These people receive the recommendation to receive an Implantable Cardioverter Defibrillator ("ICD"). The ICD is surgically implanted in the chest, and continuously monitors the person's electrocardiogram ("ECG"). If certain types of heart arrhythmias are detected, then the ICD delivers an electric shock through the heart.

After being identified as having an increased risk of an SCA, and before receiving an ICD, these people are sometimes given a wearable cardiac defibrillator ("WCD") system. A wearable defibrillator system typically includes a harness, vest, or other garment for wearing by the patient. The system includes a defibrillator and external electrodes, which are attached on the inside of the harness, vest, or other garment. When the person wears the system, the external electrodes may then make good electrical contact with the person's skin, and therefore can help monitor the person's ECG. If a shockable heart arrhythmia is detected, then the defibrillator delivers the appropriate electric shock through the person's body, and thus through the heart.

### SUMMARY

The present description gives instances of wearable cardiac defibrillator (WCD) devices, systems, system components, software, and methods, the use of which may help overcome problems and limitations of the prior art.

Embodiments of a WCD system include a measurement circuit that can render a physiological input from the patient. Such WCD systems may also receive a motion detection input that reveals whether a motion event has been detected by a motion detector.

A diagnosis may be different depending on whether motion is detected. Optionally, a value becomes assigned to a motion level parameter, in response to the motion detector detecting or not detecting a motion event. Optionally, a rhythm analysis can be based on the physiological input and on the assigned value. Optionally, a rhythm analysis of the physiological input may be performed in different manners, depending on whether or not a motion event has been detected. Optionally, a different shock/no shock criterion may be applied to the rhythm analysis, depending on whether or not a motion event has been detected. The patient may receive an electrical shock according to a shock/no shock determination.

Optionally, prompts may be different depending on whether motion is detected. In addition, if a patient input is received, learning may be accomplished that can be used in later classification of detected motion events.

An advantage over the prior art is that the differing shock criteria and/or manners of rhythm analysis are more attuned to the contexts of any motion events, and thus may yield more accurate shock/no shock determinations.

### BRIEF DESCRIPTION OF THE DRAWINGS

FIG. 1 is a diagram of components of a sample wearable defibrillator system, made according to embodiments.
FIG. 2 is a diagram showing sample components of an external defibrillator, such as the one belonging in the system of FIG. 1, and which is made according to embodiments.
FIG. 3 is a conceptual diagram for illustrating embodiments where the detection of a motion event may ultimately impact a diagnosis.
FIG. 4A is a conceptual diagram for illustrating embodiments where a motion event may be classified according to what generated it.
FIG. 4B is a block diagram for illustrating sample components for classifying a motion event.
FIG. 5 is a flowchart for illustrating methods according to embodiments.
FIG. 6 is a flowchart for illustrating methods according to embodiments.
FIG. 7 is a flowchart for illustrating methods according to embodiments.
FIG. 8 is a conceptual diagram for illustrating embodiments where the detection of a motion event may ultimately impact a diagnostic rhythm analysis operation.
FIG. 9 is a flowchart for illustrating methods according to embodiments.
FIG. 10 is a conceptual diagram for illustrating embodiments where the detection of a motion event may ultimately impact prompts and learning.
FIG. 11 is a flowchart for illustrating methods according to embodiments.
FIG. 12 is a flowchart for illustrating methods according to embodiments.

### DETAILED DESCRIPTION

As has been mentioned, the present description is about wearable cardiac defibrillator ("WCD") devices, systems, system components, software, and methods. Embodiments are now described in more detail.

A wearable cardiac defibrillator (WCD) system made according to embodiments has a number of components. These components can be provided separately as modules that can be interconnected, or can be combined with other components, etc.

A component of a WCD system can be a support structure, which is configured to be worn by the patient. The support structure can be any structure suitable for wearing, such as a harness, a vest, a half-vest - for example over the left side of the torso that positions electrodes on opposite sides of the heart, one or more belts that are configured to be worn horizontally or possibly vertically over a shoulder, another garment, and so on. The support structure can be implemented in a single component, or multiple components. For example, a support structure may have a top component resting on the shoulders, for ensuring that the defibrillation electrodes will be in the right place for defibrillating, and a bottom component resting on the hips, for carrying the bulk of the weight of the defibrillator. A single component embodiment could be with a belt around at least the torso. Other embodiments could use an adhesive structure or another way for attaching to the person, without encircling any part of the body. There can be other examples.

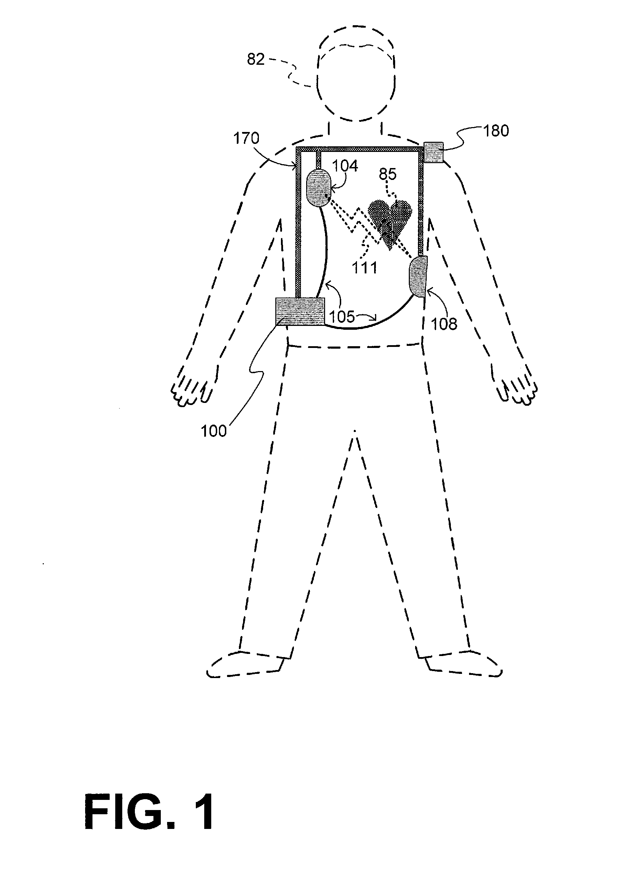

FIG. 1 depicts components of a wearable defibrillator system made according to embodiments, as it might be worn by a person 82. A person such as person 82 may also be referred to as a patient and/or wearer, since that person wears components of the wearable defibrillator system.

In FIG. 1, a generic support structure 170 is shown relative to the body of person 82, and thus also relative to his or her heart 85. Structure 170 could be a harness, a vest, a half-vest, one or more belts, or a garment, etc., as per the above. Structure 170 could be implemented in a single component, or multiple components, and so on. Structure 170 is wearable by person 82, but the manner of wearing it is not depicted, as structure 170 is depicted only generically in FIG. 1.

A wearable defibrillator system is configured to defibrillate the patient, by delivering electrical charge to the patient's body in the form of an electric shock delivered in one or more pulses. FIG. 1 shows a sample external defibrillator 100, and sample defibrillation electrodes 104, 108, which are coupled to external defibrillator 100 via electrode leads 105. Defibrillator 100 and defibrillation electrodes 104, 108 are coupled to support structure 170. As such, many of the components of defibrillator 100 can be therefore coupled to support structure 170. When defibrillation electrodes 104, 108 make good electrical contact with the body of person 82, defibrillator 100 can administer, via electrodes 104, 108, a brief, strong electric pulse 111 through the body. Pulse 111, also known as a defibrillation shock or therapy shock, is intended to go through and restart heart 85, in an effort to save the life of person 82. Pulse 111 can also be one or more pacing pulses, and so on.

A prior art defibrillator typically decides whether to defibrillate or not based on an electrocardiogram ("ECG") signal of the patient. However, defibrillator 100 can defibrillate, or not defibrillate, also based on other inputs.

The wearable defibrillator system may optionally include an outside monitoring device 180. Device 180 is called an "outside" device because it is provided as a standalone device, for example not within the housing of defibrillator 100. Device 180 can be configured to monitor at least one local parameter. A local parameter can be a parameter of patient 82, or a parameter of the wearable defibrillation system, or a parameter of the environment, as will be described later in this document.

Optionally, device 180 is physically coupled to support structure 170. In addition, device 180 can be communicatively coupled with other components, which are coupled to support structure 170. Such communication can be a communication module, as will be deemed applicable by a person skilled in the art in view of this disclosure.

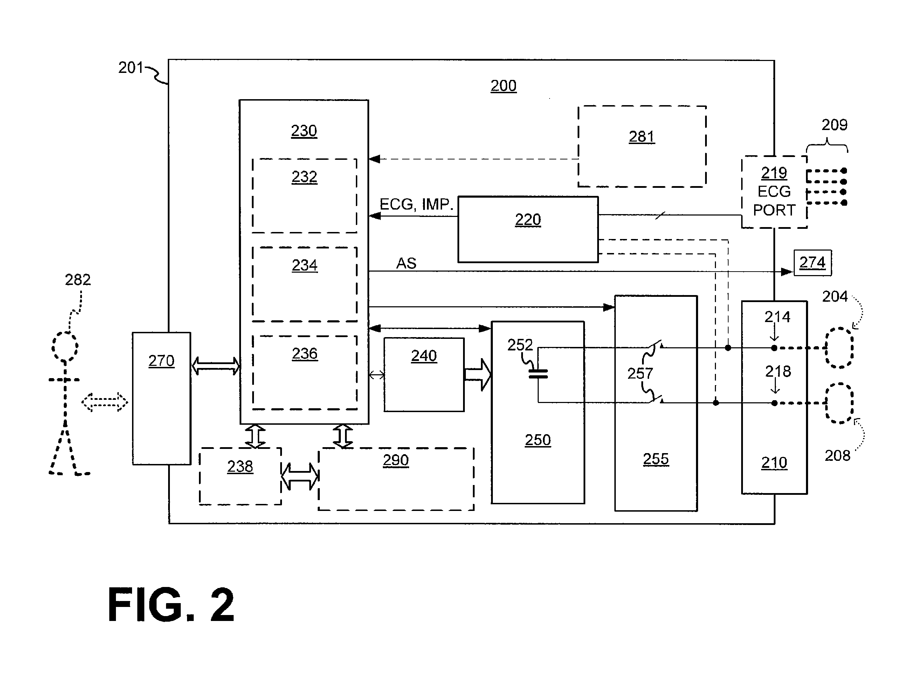

FIG. 2 is a diagram showing components of an external defibrillator 200, made according to embodiments. These components can be, for example, included in external defibrillator 100 of FIG. 1. The components shown in FIG. 2 can be provided in a housing 201, which is also known as casing 201.

External defibrillator 200 is intended for a patient who would be wearing it, such as person 82 of FIG. 1. Defibrillator 200 may further include a user interface 270 for a user 282. User 282 can be patient 82, also known as wearer 82. Or user 282 can be a local rescuer at the scene, such as a bystander who might offer assistance, or a trained person. Or, user 282 might be a remotely located trained caregiver in communication with the wearable defibrillator system.

User interface 270 can be made in any number of ways. User interface 270 may include output devices, which can be visual, audible or tactile, for communicating to a user. An output device can be configured to output a warning, which warns or instructs the patient or a bystander to do something. For example, interface 270 may include a screen, to display what is detected and measured, provide visual feedback to rescuer 282 for their resuscitation attempts, and so on. Interface 270 may also include a speaker, to issue voice prompts, etc. Sounds, images, vibrations, and anything that can be perceived by user 282 can also be called human perceptible indications. User interface 270 may also include input devices for receiving inputs from users. Such input devices may include various controls, such as pushbuttons, keyboards, touchscreens, a microphone, and so on. An input device can be a cancel switch, which is sometimes called a "live-man" switch. In some embodiments, actuating the cancel switch within a preset time after a prompt with a warning can prevent the impending delivery of a shock. In addition, discharge circuit 255 can be controlled by processor 230, or directly by user 282 via user interface 270, and so on.

Defibrillator 200 may include an internal monitoring device 281. Device 281 is called an "internal" device because it is incorporated within housing 201. Monitoring device 281 can monitor patient parameters, patient physiological parameters, system parameters and/or environmental parameters, all of which can be called patient data. In other words, internal monitoring device 281 can be complementary or an alternative to outside monitoring device 180 of FIG. 1. Allocating which of the system parameters are to be monitored by which monitoring device can be done according to design considerations.

Patient physiological parameters include, for example, those physiological parameters that can be of any help in detecting by the wearable defibrillation system whether the patient is in need of a shock, plus optionally their medical history and/or event history. Examples of such parameters include the patient's ECG, blood oxygen level, blood flow, blood pressure, blood perfusion, pulsatile change in light transmission or reflection properties of perfused tissue, heart sounds, heart wall motion, breathing sounds and pulse. Accordingly, the monitoring device could include a perfusion sensor, a pulse oximeter, a Doppler device for detecting blood flow, a cuff for detecting blood pressure, an optical sensor, illumination detectors and perhaps sources for detecting color change in tissue, a motion sensor, a device that can detect heart wall movement, a sound sensor, a device with a microphone, an SpO2 sensor, and so on. Pulse detection is taught at least in Physio-Control's US Patent No. 8,135,462, which is hereby incorporated by reference in its entirety. In addition, a person skilled in the art may implement other ways of performing pulse detection.

In some embodiments, the local parameter is a trend that can be detected in a monitored physiological parameter of patient 82. A trend can be detected by comparing values of parameters at different times. Parameters whose detected trends can particularly help a cardiac rehabilitation program include: a) cardiac function (e.g. ejection fraction, stroke volume, cardiac output, etc.); b) heart rate variability at rest or during exercise; c) heart rate profile during exercise and measurement of activity vigor, such as from the profile of an accelerometer signal and informed from adaptive rate pacemaker technology; d) heart rate trending; e) perfusion, such as from SpO2 or CO2; f) respiratory function, respiratory rate, etc.; g) motion, level of activity; and so on. Once a trend is detected, it can be stored and/or reported via a communication link, along perhaps with a warning. From the report, a physician monitoring the progress of patient 82 will know about a condition that is either not improving or deteriorating.

Patient state parameters include recorded aspects of patient 82, such as motion, posture, whether they have spoken recently plus maybe also what they said, and so on, plus optionally the history of these parameters. Or, one of these monitoring devices could include a location sensor such as a Global Positioning System (GPS) location sensor. Such a sensor can about the location, plus a speed can be detected as a rate of change of location over time. Many motion detectors output a motion signal that is indicative of the motion of the detector, and thus of the patient's body. Patient state parameters can be very helpful in narrowing down the determination of whether SCA is indeed taking place.

A wearable cardiac defibrillator (WCD) system made according to embodiments may include a motion detector. In embodiments, a motion detector can be implemented within outside monitoring device 180 or inside monitoring device 281. Such a motion detector can be configured to detect a motion event. In response, the motion detector may render or generate from the detected motion event a motion detection input that can be received by a subsequent device or functionality. A motion event can be defined as is convenient, for example a change in motion from a baseline motion or rest, etc. Such a motion detector can be made in many ways as is known in the art, for example by using an accelerometer. In some embodiments, the motion detector itself is not part of the WCD system, but it can communicate the motion detection input to a suitable part of the WCD system, for example wirelessly. In embodiments, it can be determined whether the motion detector detected a motion event. Determining can be by judging from a motion detection input from the motion detector, or the absence of such a motion detection input, depending on the final configuration.

System parameters of a wearable defibrillation system can include system identification, battery status, system date and time, reports of self-testing, records of data entered, records of episodes and intervention, and so on.

Environmental parameters can include ambient temperature and pressure. A humidity sensor may provide information as to whether it is likely raining. Presumed patient location could also be considered an environmental parameter. The patient location could be presumed if monitoring device 180 or 281 includes a GPS location sensor as per the above.

Defibrillator 200 typically includes a defibrillation port 210, such as a socket in housing 201. Defibrillation port 210 includes electrical nodes 214, 218. Leads of defibrillation electrodes 204, 208, such as leads 105 of FIG. 1, can be plugged in defibrillation port 210, so as to make electrical contact with nodes 214, 218, respectively. It is also possible that defibrillation electrodes 204, 208 are connected continuously to defibrillation port 210, instead. Either way, defibrillation port 210 can be used for guiding, via electrodes, to the wearer the electrical charge that has been stored in energy storage module 250. The electric charge will be the shock for defibrillation, pacing, and so on.

Defibrillator 200 may optionally also have an ECG port 219 in housing 201, for plugging in sensing electrodes 209, which are also known as ECG leads. It is also possible that Sensing electrodes 209 can be connected continuously to ECG port 219, instead. Sensing electrodes 209 can help sense an ECG signal, e.g. a 12-lead signal, or a signal from a different number of leads, especially if they make good electrical contact with the body of the patient. Sensing electrodes 209 can be attached to the inside of support structure 170 for making good electrical contact with the patient, similarly as defibrillation electrodes 204, 208.

Optionally a wearable defibrillator system according to embodiments also includes a fluid that it can deploy automatically between the electrodes and the patient skin. The fluid can be conductive, such as by including an electrolyte, for making a better electrical contact between the electrode and the skin. Electrically speaking, when the fluid is deployed, the electrical impedance between the electrode and the skin is reduced. Mechanically speaking, the fluid may be in the form of a low-viscosity gel, so that it does not flow away, after it has been deployed. The fluid can be used for both defibrillation electrodes 204, 208, and sensing electrodes 209.

The fluid may be initially stored in a fluid reservoir, not shown in FIG. 2, which is can be coupled to the support structure. In addition, a wearable defibrillator system according to embodiments further includes a fluid deploying mechanism 274. Fluid deploying mechanism 274 can be configured to cause at least some of the fluid to be released from the reservoir, and be deployed near one or both of the patient locations, to which the electrodes are configured to be attached to the patient. In some embodiments, fluid deploying mechanism 274 is activated responsive to receiving activation signal AS from processor 230, prior to the electrical discharge.

Defibrillator 200 also includes a measurement circuit 220. Measurement circuit 220 receives physiological signals of the patient from ECG port 219, if provided. Even if defibrillator 200 lacks ECG port 219, measurement circuit 220 can obtain physiological signals through nodes 214, 218 instead, when defibrillation electrodes 204, 208 are attached to the patient. In these cases, the patient's ECG signal can be sensed as a voltage difference between electrodes 204, 208. Plus, impedance between electrodes 204, 208 and/or the connections of ECG port 219 can be sensed. Sensing the impedance can be useful for detecting, among other things, whether these electrodes 204, 208 and/or sensing electrodes 209 are not making good electrical contact with the patient's body. These patient physiological signals can be sensed, when available. Measurement circuit 220 can then render or generate information about them as physiological inputs, data, other signals, etc. More strictly speaking, the information rendered by measurement circuit 220 is output from it, but this information can be called an input because it is received by a subsequent device or functionality as an input.

Defibrillator 200 also includes a processor 230. Processor 230 may be implemented in any number of ways. Such ways include, by way of example and not of limitation, digital and/or analog processors such as microprocessors and digital-signal processors (DSPs); controllers such as microcontrollers; software running in a machine; programmable circuits such as Field Programmable Gate Arrays (FPGAs), Field-Programmable Analog Arrays (FPAAs), Programmable Logic Devices (PLDs), Application Specific Integrated Circuits (ASICs), any combination of one or more of these, and so on.

Processor 230 can be considered to have a number of modules. One such module can be a detection module 232. Detection module 232 can include a ventricular fibrillation ("VF") detector. The patient's sensed ECG from measurement circuit 220, which can be available as physiological inputs, data, or other signals, may be used by the VF detector to determine whether the patient is experiencing VF. Detecting VF is useful, because VF results in SCA. Detection module 232 can also include a ventricular tachycardia ("VT") detector, and so on.

Another such module in processor 230 can be an advice module 234, which generates advice for what to do. The advice can be based on outputs of detection module 232. There can be many types of advice according to embodiments. In some embodiments, the advice is a shock/no shock determination that processor 230 can make, for example via advice module 234. The shock/no shock determination can be made by executing a stored Shock Advisory Algorithm. A Shock Advisory Algorithm can make a shock/no shock determination from one or more of ECG signals that are captured according to embodiments, and determining whether a shock criterion is met. The determination can be made from a rhythm analysis of the captured ECG signal or otherwise.

In some embodiments, when the decision is to shock, an electrical charge is delivered to the patient. In some embodiments, processor 230 can be configured to control discharge circuit 255 to discharge electrical charge stored in energy storage module 250 through the patient. Delivering the electrical charge is also known as discharging. Shocking can be for defibrillation, pacing, and so on.

Processor 230 can include additional modules, such as other module 236, for other functions. In addition, if internal monitoring device 281 is indeed provided, it may be operated in part by processor 230, etc.

Defibrillator 200 optionally further includes a memory 238, which can work together with processor 230. Memory 238 may be implemented in any number of ways. Such ways include, by way of example and not of limitation, volatile memories, nonvolatile memories (NVM), read-only memories (ROM), random access memories (RAM), magnetic disk storage media, optical storage media, smart cards, flash memory devices, any combination of these, and so on. Memory 238 is thus a non-transitory storage medium. Memory 238, if provided, can include programs for processor 230, which processor 230 may be able to read, and execute. More particularly, the programs can include sets of instructions in the form of code, which processor 230 may be able to execute upon reading. Executing is performed by physical manipulations of physical quantities, and may result in the functions, processes, actions and/or methods to be performed, and/or the processor to cause other devices or components or blocks to perform such functions, processes, actions and/or methods. The programs can be operational for the inherent needs of processor 230, and can also include protocols and ways that decisions can be made by advice module 234. In addition, memory 238 can store prompts for user 282, if they are a local rescuer. Moreover, memory 238 can store data. The data can include patient data, system data and environmental data, for example as learned by internal monitoring device 281 and outside monitoring device 180. The data can be stored in memory 238 before it is transmitted out of defibrillator 200, or stored there after it is received by it.

Defibrillator 200 may also include a power source 240. To enable portability of defibrillator 200, power source 240 typically includes a battery. Such a battery is typically implemented as a battery pack, which can be rechargeable or not. Sometimes a combination is used of rechargeable and non-rechargeable battery packs. Other embodiments of power source 240 can include an AC power override, for where AC power will be available, an energy storage capacitor, and so on. In some embodiments, power source 240 is controlled by processor 230.

Defibrillator 200 additionally includes an energy storage module 250, which can thus be coupled to the support structure of the wearable system. Module 250 is where some electrical energy is stored in the form of an electrical charge, when preparing it for sudden discharge to administer a shock. Module 250 can be charged from power source 240 to the right amount of energy, as controlled by processor 230. In typical implementations, module 250 includes a capacitor 252, which can be a single capacitor or a system of capacitors, and so on. As described above, capacitor 252 can store the energy in the form of electrical charge, for delivering to the patient.

Defibrillator 200 moreover includes a discharge circuit 255. Circuit 255 can be controlled via processor 230 or user interface 270. When so controlled, circuit 255 can permit the energy stored in module 250 to be discharged to nodes 214, 218, and from there also to defibrillation electrodes 204, 208. Circuit 255 can include one or more switches 257. Switches 257 can be made in a number of ways, such as by an H-bridge, and so on.

Defibrillator 200 can optionally include a communication module 290, for establishing one or more wired or wireless communication links with other devices of other entities, such as a remote assistance center, Emergency Medical Services (EMS), and so on. Module 290 may also include an antenna, portions of a processor, and other sub-components as may be deemed necessary by a person skilled in the art. This way, data and commands can be communicated, such as patient data, event information, therapy attempted, CPR performance, system data, environmental data, and so on.

Defibrillator 200 can optionally include other components.

Returning to FIG. 1, in embodiments, one or more of the components of the shown WCD system may have been customized for patient 82. This customization may include a number of aspects. For instance, support structure 170 can be fitted to the body of patient 82. For another instance, baseline physiological parameters of patient 82 can be measured, such as the heart rate of patient 82 while resting, while walking, etc. In addition, outputs of monitoring devices 180, 281 can be recorded while patient 82 is known to be walking, for example during the fitting. Such baseline physiological parameters can be used to customize the WCD system, in order to make its diagnoses more accurate, since bodies behave differently. For example, they can be stored in memory 238, and so on.

A programming interface can be made according to embodiments, which receives such measured baseline physiological parameters. Such a programming interface may input automatically in the WCD system the baseline physiological parameters, along with other data.

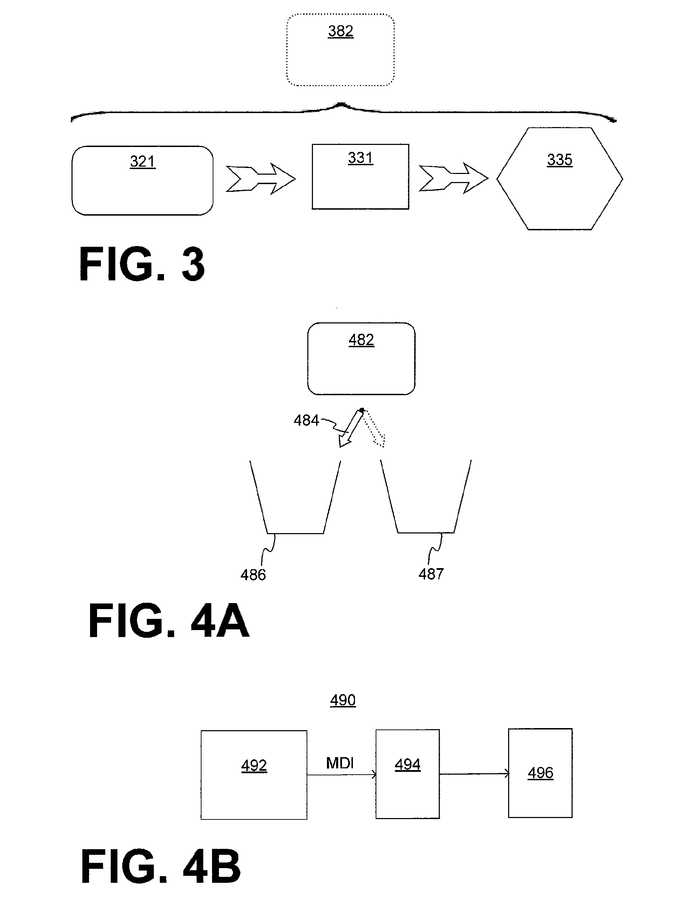

In embodiments, a diagnosis by a WCD system may be different, depending on whether a motion event was detected or not. FIG. 3 is a conceptual diagram for illustrating embodiments where the detection of a motion event may ultimately impact diagnostic operations of a WCD system. A physiological input 321 can be received, a rhythm analysis 331 can be performed, and a shock/no shock recommendation 335 can be arrived at from rhythm analysis 331. A possible motion event 382 is shown in dotted lines, because it may be detected or not by a motion detector. If it is present, it may affect physiological input 321, or rhythm analysis 331, or shock/no shock recommendation 335, or any combination of these, as will be seen later in this document.

In embodiments, if a motion event has been detected, it can be further optionally classified in one or more categories. Examples are now described.

FIG. 4A is a conceptual diagram for illustrating embodiments where a motion event may be classified according to what generated it. A motion event 482 may be classified according to an arrow 484 in one of two categories 486, 487, shown in FIG. 4A as buckets 486, 487. Bucket 486 is for motion events that are regarded as ambient, which could be due to a number of reasons, such as the patient being transported by a vehicle. Bucket 487 is for motion events that are regarded as conscious-patient generated, meaning that the patient is moving around, and therefore not suffering from SCA. More categories can be defined; each category can be subdivided in subcategories; and so on.

There are a number of ways of making a classification such as the classification of FIG. 4A according to embodiments. For example, motion event 482 can be classified in one of buckets 486, 487 if it meets certain criteria. Additionally, motion event 482 can be classified in the other bucket as a default, if it does not meet certain criteria.

There can be a number of such criteria according to embodiments. For example, a motion event can be classified from a motion detection input that was generated by a motion detector that detected the motion event. The motion detection input can be analyzed. For instance, an aspect of this motion detection input can be similar enough, i.e. within a tolerance threshold, to a reference input that has been stored in memory 238 and classified as ambient, or conscious-patient generated, or otherwise. The reference input can be for detecting a set of everyday activities, such as washing dishes and vacuuming. These activities may be detected by comparing a predetermined set of signal values, such as acceleration parameters from an accelerometer, with predetermined thresholds that are characteristic of those activities. The aspect can be an amplitude aspect or a time profile of a signal, and so on.

In some embodiments the classification may be more specific. For example, a WCD system might specifically detect walking and/or running vs other kinds of motion. There is a periodicity to walking and running that is less likely to be confused with vehicle motion than a simple motion detector. In addition, walking and running are very common persistent activities, so precluding unnecessary shocks during these activities could be beneficial. Other types of patient motion (e.g. gardening, washing dishes, vacuuming) might not be covered, but that drawback may be minor compared to the reduced risk of a false negative shock recommendation due to vehicle motion. A walking/running detection algorithm could (for example) look for a periodicity in the range of 1 - 3Hz with a vertical-axis RMS acceleration greater than a threshold value. Accordingly, the motion event can be classified as walking/running vs other based on the patient activity that likely generated it.

In some embodiments, the motion detector includes an accelerometer. The accelerometer may be a one-axis accelerometer measuring, for example, an acceleration in the vertical direction, or it may be a three-axis accelerometer. The accelerometer can be configured to output a motion detection signal, such as the accelerometer signal. In such embodiments, the motion event may become classified depending on the motion detection signal, for example by comparing the signal amplitude to a threshold. A motion detection algorithm could include the accelerometer mean value, median value, maximum or minimum value, peak-peak value, the RMS value, the signal variance, standard deviation, or other calculation. The signal may be high or low-pass filtered. It may be median filtered. It may be adaptively filtered. The algorithm could include a measurement of the skewness, kurtosis or entropy. It could include measurements based on an autocorrelation of the signal, a frequency transformation of the signal, a wavelet transformation of the signal, or a Hilbert transformation of the signal.

If an accelerometer with more than one measurement axis is included, then combinations of movements may improve the accuracy of the motion detection algorithm compared to a single measurement. One way of combining features is to form a linear combination of the form: Score = A * Featurel + B * Feature2, where A and B are constants, and Feature1 and Feature 2 are measurements made on the accelerometer signal. A score value greater than a threshold would indicate that motion was detected.

The duration of the activity may also be considered as part of the motion detection algorithm. In general, transient artifacts are less of a concern than persistent artifacts. In this context, artifacts that last less than 5 seconds are unlikely to trigger an incorrect rhythm analysis. As such, there is little need to detect motion that lasts less than 5 seconds. The motion detection algorithm can therefore avoid false positive motion indications by only flagging motion for signals that persist for more than a threshold amount of time (e.g. 5 seconds).

In some embodiments the motion detector may not just assess patient motion but may also consider the patient orientation (i.e. is the patient upright or lying down). One way of determining the patient orientation is to use a 3-axis accelerometer to measure the force of gravity. If a vertically-oriented accelerometer measures a sustained acceleration greater than a threshold (e.g. 0.5 G) then the patient would be considered to be upright. In this embodiment a patient who is upright would receive a different rhythm analysis than one who is not upright.

In some embodiments the motion detector includes a gyroscope that is configured to output a motion detection signal. In such embodiments the motion event may become classified depending on the measured rotational velocity compared to a predetermined threshold.

In some embodiments, the motion detector includes a first accelerometer and a second accelerometer that can be moved with respect to the first accelerometer. These two, or even more than two accelerometers could be within movable parts of the WCD system. In addition, inputs may be received from accelerometers on the patient that are independent of the WCD system, such as those in a smart phone, smart watch, or other device.

These two or more accelerometers may be configured to render respective motion detection inputs from detecting a motion event, such as motion detection signals. In such embodiments, the motion event may become classified by comparing the respective motion detection inputs. Other embodiments are described later in this document. If the signals from an accelerometer in the WCD system and in a smart watch are similar, it is likely that the activity is ambient motion. If the signals are different, then the activity is likely to be conscious-patient generated.

There are many possible embodiments for motion detection inputs, which can be implemented via analog circuit designs, digital processing, and so on. An example is now described.

FIG. 4B is a block diagram for illustrating a set 490 of components for classifying a motion event. One or more of the components of set 490 could be provided in, or distributed among, outside monitoring device 180, inside monitoring device 281 and processor 230, and so on.

Set 490 includes a motion detector 492, which could be a 1-axis accelerometer or a 3-axis accelerometer. Motion detector 492 generates a motion detection input MDI.

Set 490 also optionally includes an Analog-to-Digital Converter (ADC) 494, for receiving motion detection input MDI, and generating digital values for it. Of course, in some embodiments, the output of ADC 494 is considered to be the motion detection input, and so on. Thus, ADC is used in classifying in a number of embodiments.

Set 490 further includes a stage 496 that can include filters such as high pass filters, low pass filters, band pass filters, and processing that implements detection and classification of motion events from their motion detection input. The processing can be implemented with detection algorithms, classification algorithms, and so on. Stage 496 can output values that indicate how a motion event is classified, in many possible sets of categories. One set of categories is what was described with reference to FIG. 4A; another set may include "Falling Detection", and so on.

The devices and/or systems mentioned in this document perform functions, processes and/or methods. These functions, processes and/or methods may be implemented by one or more devices that include logic circuitry. Such a device can be alternately called a computer, and so on. It may be a standalone device or computer, such as a general purpose computer, or part of a device that has one or more additional functions. The logic circuitry may include a processor and non-transitory computer-readable storage media, such as memories, of the type described elsewhere in this document. Often, for the sake of convenience only, it is preferred to implement and describe a program as various interconnected distinct software modules or features. These, along with data are individually and also collectively known as software. In some instances, software is combined with hardware, in a mix called firmware.

Moreover, methods and algorithms are described below. This detailed description includes flowcharts, display images, algorithms, and symbolic representations of program operations within at least one computer readable medium. An economy is achieved in that a single set of flowcharts is used to describe both programs, and also methods. So, while flowcharts described methods in terms of boxes, they also concurrently describe programs.

Methods are now described.

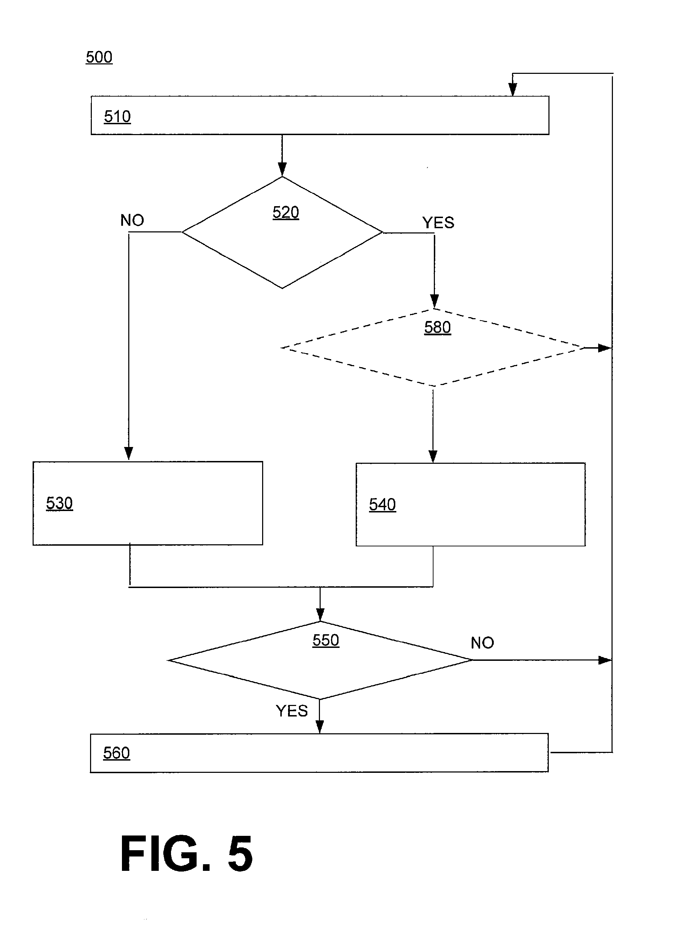

FIG. 5 shows a flowchart 500 for describing methods according to embodiments. The methods of flowchart 500 may also be practiced by embodiments described elsewhere in this document.

According to an operation 510, a physiological input is received.

According to another operation 520, it may be determined whether or not a motion event was detected. In embodiments, operation 520 may be implemented by determining whether a motion detector detected a motion event by a motion detection input from the motion detector, or an absence of such a motion detection input. If a motion event were not detected at operation 520, then according to another operation 530, a rhythm analysis may be performed based on the physiological input in a first manner. If a motion event were detected at operation 520, then according to another operation 540 a rhythm analysis may be performed based on the physiological input in a second manner. The second manner can be different from the first manner. In some embodiments, operation 540 is to assume that defibrillation will not be required, for example as indicated by operation 580 if the detected motion event becomes classified as conscious-patient generated.

The two different manners of performing a rhythm analysis may be implemented in any number of ways. For example, the physiological input can be derived from an ECG signal. In the first manner the ECG signal can be high-pass filtered at a first cutoff frequency, while in the second manner the ECG signal can be high-pass filtered at a second cutoff frequency different from the first cutoff frequency. For instance, 3Hz high-pass filter might be used for operation 530, but a 6Hz high pass filter may be used for operation 540. The 6Hz high-pass filter makes the algorithm less likely to detect some kinds of Ventricular Tachycardia and VF, but it also removes more of the artifact.

According to another operation 550, it can be determined whether a shock criterion is met. The determination may be made from the rhythm analysis that has been performed, in other words, the rhythm analysis of either operation 530 or operation 540. If at operation 550 the answer is no, execution may return to operation 510 or another operation. If at operation 550 the answer is yes then, according to another operation 560, the discharge circuit may be caused to discharge the electrical charge through the patient. Then execution may return to operation 510 or another operation.

Within flowchart 500, another operation 580 may be optionally executed. According to operation 580, the detected motion event may be classified, for example as ambient or conscious-patient generated. If the latter, then execution may bypass any one of operations 540, 550, 560, and ultimately the discharge circuit would not be caused to discharge.

The classification of operation 580 may be performed in any number of ways. Some of these ways were described above. Additional examples are now described.

In some embodiments, the measurement circuit includes an impedance detector that can be configured to generate an impedance sense input. If a motion event has been detected at operation 520, the motion event may be classified as ambient or conscious-patient generated according to the impedance sense input. These embodiments may work because conscious-patient generated motion may tend to produce activity on the accelerometer and on the impedance signal, while ambient motion will tend to show up mainly on the accelerometer.

In some embodiments, the WCD system further includes a GPS location sensor that may be configured to generate a location sense input. If a motion event has been detected at operation 520, the motion event may be classified as ambient or conscious-patient generated according to the location sense input. These embodiments may work because a GPS location sensor can capture movement in a vehicle for transportation, and help classify it as ambient activity. Such vehicle may include airplanes, trains, automobiles, motor bicycles, bicycles, and vessels. GPS has advantages over an accelerometer because it can confirm the wider context of a vehicle in motion, which helps because the vehicle motion might not be uniform. Also, accelerometer drift makes absolute velocity measurements difficult, especially over time. For example, an automobile driven in a city may stop and go, accelerating to some speeds, then slow down and stop. Even while stopped, it may generate an underlying vibration. These activities may produce signals that seem different to an accelerometer, while GPS can confirm the wider context that the patient is in a moving vehicle.

In some embodiments the accuracy of motion detection may be enhanced or augmented using a cell phone signal or a Wi-Fi signal. The WCD system may include a cellphone signal sensor that can detect an ambient cellphone signal, or a WiFi signal sensor that can detect an ambient WiFi signal. These signals could be used instead of a GPS signal or in addition to a GPS signal to classify the motion event and/or confirm the wider context that the patient is in a moving vehicle.

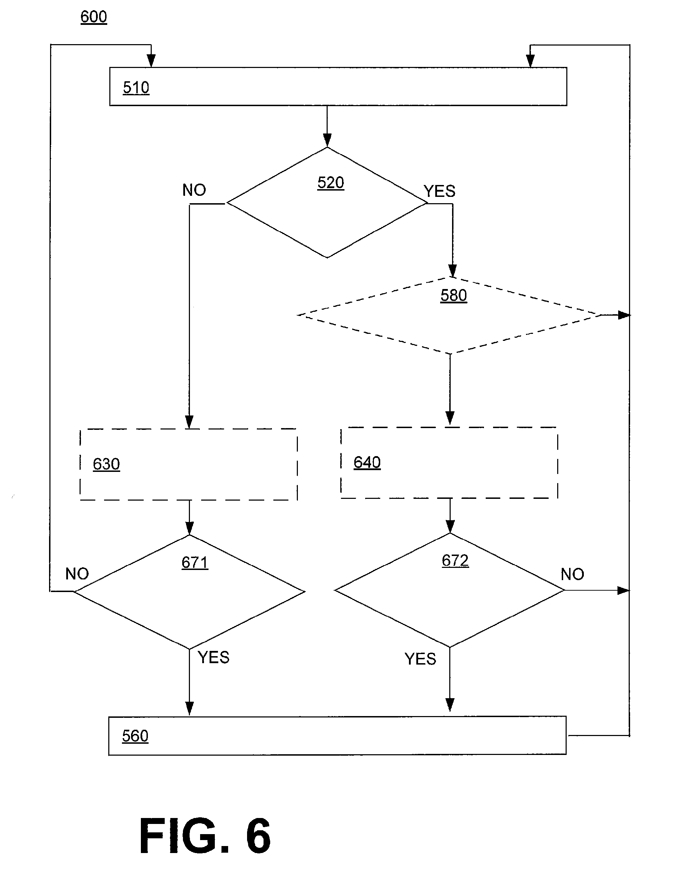

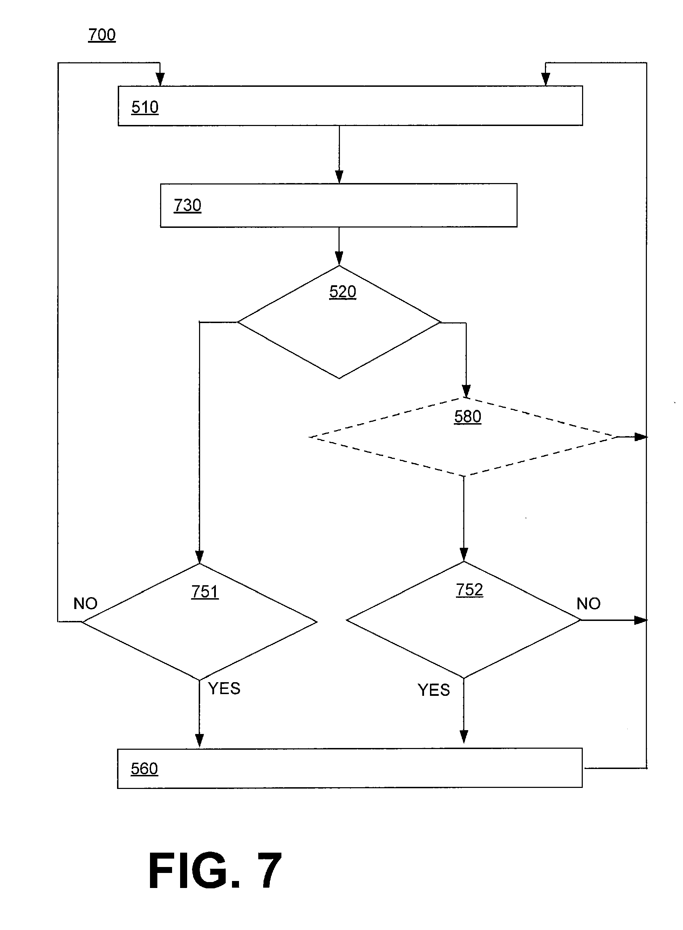

In some embodiments, the shock criterion is different. There are a number of ways this can be performed. Two examples are now described, with reference to FIG. 6 and FIG. 7.

FIG. 6 shows a flowchart 600 for describing methods according to embodiments. The methods of flowchart 600 may also be practiced by embodiments described elsewhere in this document. It will be recognized that flowchart 600 includes operations 510, 520, 560 and 580 that were described above.

According to an operation 671, it is determined from the physiological input of operation 510 whether a still shock criterion is met. If it is, execution may proceed to operation 560, else it may return to 510 or another operation.

According to another operation 672, it is determined from the physiological input of operation 510 whether a moving shock criterion is met. If it is, execution may proceed to operation 560, else it may return to 510 or another operation.

The moving shock criterion of operation 672 may be different from the still shock criterion of operation 671. In this first sample embodiment, the determination of whether the still shock criterion is met may be made by performing a rhythm analysis in a first manner according to operation 630, while the determination of whether the moving shock criterion is met may be made by performing a rhythm analysis based on the physiological input in a second manner according to operation 640. Operations 630 and 640 may be as described above with reference to operations 530 and 540. Again, the second manner may be different from the first manner; the physiological input may be derived from an ECG signal that is high-pass filtered at different cutoff frequencies; and so on.

FIG. 7 shows a flowchart 700 for describing methods according to embodiments. The methods of flowchart 700 may also be practiced by embodiments described elsewhere in this document. It will be recognized that flowchart 700 includes operations 510, 520, 560 and 580 that were described above.

According to an operation 730, a rhythm analysis is performed. Operation 730 may be performed at the instant shown, or later in flowchart 700.

According to an operation 751, it is determined from the physiological input of operation 510 whether a still shock criterion is met, based on the rhythm analysis of operation 730. If it is, execution may proceed to operation 560, else it may return to 510 or another operation.

According to another operation 752, it is determined from the physiological input of operation 510 whether a moving shock criterion is met, based on the rhythm analysis of operation 730. If it is, execution may proceed to operation 560, else it may return to 510 or another operation.

The moving shock criterion of operation 752 may be different from the still shock criterion of operation 751. In this second sample embodiment a rhythm analysis may be performed from the physiological input, and a shock advice parameter can be generated from the rhythm analysis. The still shock criterion can be that the shock advice parameter has a value greater than a first threshold, while the moving shock criterion can be that the shock advice parameter has a value greater than a second threshold. The second threshold can be different from the first threshold. For example, the shock advice parameter can be a number from zero to 10, to indicate how likely it is that patient needs to be shocked. In such cases, the still shock criterion can be ">5", i.e. if no motion event is detected at operation 671, and the moving shock criterion can be ">7", i.e. if a motion event is detected at operation 672.

As mentioned above, in embodiments, the motion event may be classified as walking/running vs other. For diagnosing, there can be different criteria that are to be met, or altogether different algorithms, depending on the classification.

In some embodiments, the rhythm analysis may be performed in a first manner if no motion event has been detected, in a second manner if the motion event has become classified as walking/running, and in a third manner if the motion event has become classified as other. The third manner can be different from the second manner and possibly also from the first manner.

In some embodiments, the moving criterion is different, depending on whether the motion event has become classified as walking/running or other. Combinations may be also implemented.

In some of the previous examples, diagnostic algorithms were executed that may have been different at some point depending on whether or not a motion event is detected. Such is not necessarily always the case, and a single algorithm may be executed according to embodiments, which incorporates a parameter for motion. An example is now described.

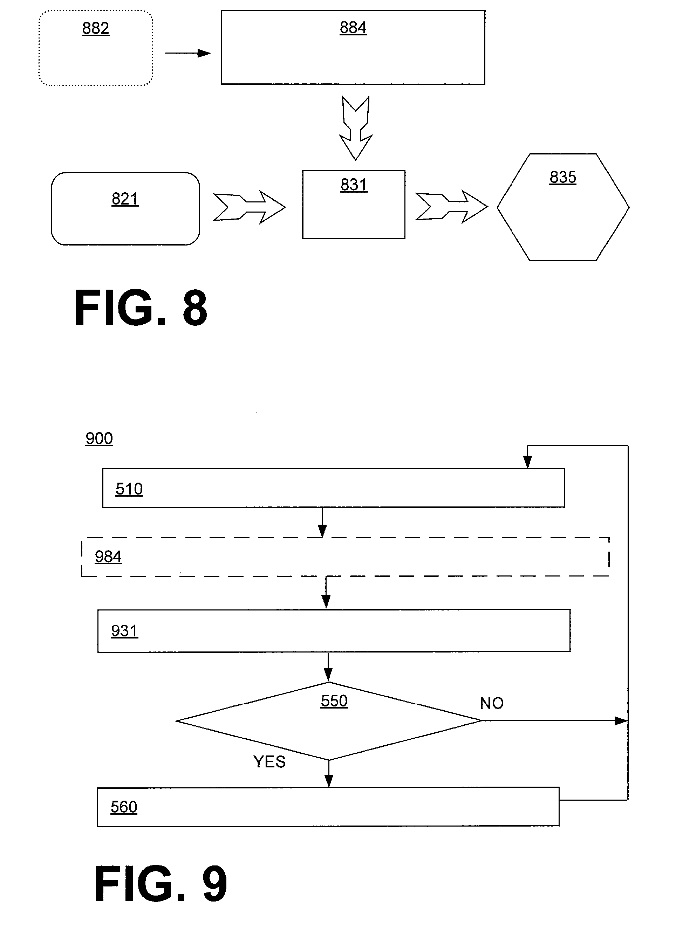

FIG. 8 is a conceptual diagram for illustrating embodiments where the detection of a motion event may ultimately impact a rhythm analysis operation. A physiological input 821 is received. In addition, by an operation 884 a value, such as a numerical value, can become assigned to a motion level parameter in response to a motion detector detecting a possible motion event 882, or not detecting a motion event. The value can become assigned by evaluation circuitry of the motion detector, or by the processor. The value could have a magnitude reflecting a level of the detected motion event. For example, the value can be a large number for a large detected amplitude of motion averaged over some time, zero if no motion event is detected, and so on.

A rhythm analysis 831 can be performed from physiological input 821, and also accounting from the value of the motion level parameter. A shock/no shock recommendation 835 can be arrived at from rhythm analysis 831.

FIG. 9 shows a flowchart 900 for describing methods according to embodiments. The methods of flowchart 900 may also be practiced by embodiments described elsewhere in this document. It will be recognized that flowchart 900 includes operations 510, 550 and 560 that were described above.

According to another, optional operation 984, a value, such as a numerical value, can become assigned to a motion level parameter in response to a possible detected motion event. Even if there is no motion event, the motion level parameter can have a suitable value, perhaps zero.

According to another operation 931, a rhythm analysis may be performed. The rhythm analysis may be based on the physiological input and on the assigned value of the motion level parameter.

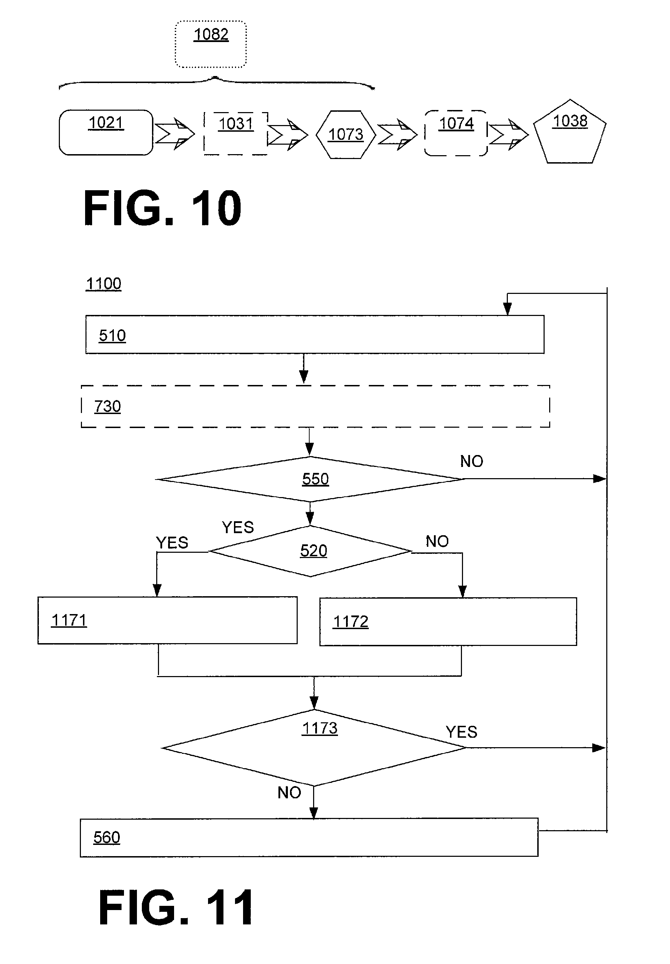

In embodiments, prompts by a WCD system may be different, depending on whether a motion event was detected or not. FIG. 10 is a conceptual diagram for illustrating embodiments where the detection of a motion event may ultimately impact prompts of a WCD system.

In FIG. 10, a physiological input 1021 may be received, a rhythm analysis 1031 can be performed, and a patient prompt 1073 may be generated as a result. A possible motion event 1082 is shown in dotted lines, because it may be detected or not by a motion detector. If it is detected, it may affect prompt 1073, as will be seen later in this document. In addition, if a patient input 1074 is received in response to the prompts, such as from a cancel switch, learning 1038 may be accomplished that can be used in later classification of detected motion events. Learning 1038 can be by storing appropriate data in memory 238.

FIG. 11 shows a flowchart 1100 for describing methods according to embodiments. The methods of flowchart 1100 may also be practiced by embodiments described elsewhere in this document. Processor 230 can cause an output device of user interface 270 to output warnings. It will be recognized that flowchart 1100 includes operations 510, optionally 730, 550, 520 and 560 that were described above. At operation 520, a motion event can be detected by a motion detection input from the motion detector, or absence of such an input.

If at operation 520 a motion event has been detected then, according to another operation 1171, a first prompt may be output by an output device of the WCD system. The first prompt can instruct the patient to actuate the cancel switch, and need not be of an alarming nature. For example, the output device can be a speaker that says something like, "Press the cancel button to confirm patient activity." In this case the response button is simply a confirmation that the patient is active and not in need of therapy.

If at operation 520 no motion event has been detected then, according to another operation 1172, a second prompt may be output by an output device of the WCD system. The second prompt can instruct the patient to actuate the cancel switch, but the second prompt can be different from the first prompt at least in part. For example, the second prompt can be alarming rather than reassuring. For instance, a speaker could say something like, "Shockable rhythm detected! Press the cancel button now to avoid therapy!" In this case it is assumed that the patient is not conscious and is in need of a shock. The second prompt therefore may convey the urgency of the situation.

At another operation 1173, it is determined whether the cancel switch has been actuated, within a preset time after one of the first prompt and the second prompt has been outputted. If not, the operation 560 takes place. If yes, then operation 560 can be bypassed. The preset time can also be called a react interval, for example as described in copending US Patent Application SN 14/014,987, which is incorporated herein by reference.

In some embodiments, the WCD system is capable of learning. For example, the discharge circuit might not be caused to discharge the electrical charge though the patient, if the motion detection input indicates that the motion event is continuing. An aspect of the motion detection event could be stored in a memory such as memory 238, for detecting that the motion is continuing. So, even if another rhythm analysis is performed, and it is determined from the other rhythm analysis that the shock criterion is met.

In other words, the WCD system's behavior may continue for a period of time, or it may continue until the motion is stopped, after which time it could revert back to the normal analysis. In such embodiments, a person walking their dog may need to press the cancel button once to cancel the shock therapy, but would not need to continue pushing it for as long as they were walking.

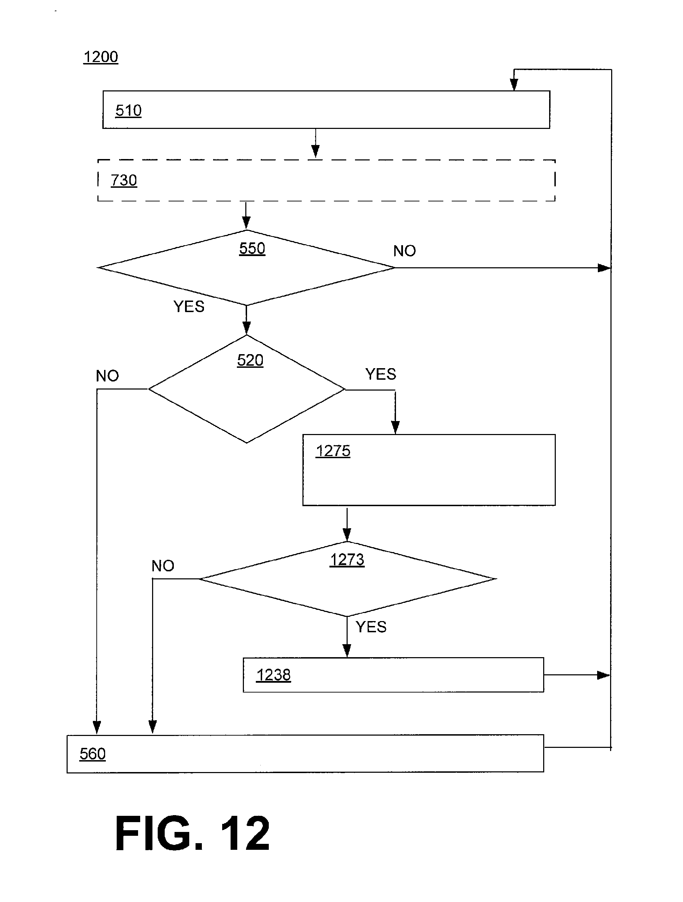

FIG. 12 shows a flowchart 1200 for describing methods according to embodiments. The methods of flowchart 1200 may also be practiced by embodiments described elsewhere in this document. It will be recognized that flowchart 1200 includes operations 510, optionally 730, 550, 520, and 560 that were described above.

If at operation 520 it is determined that no motion event has been detected then execution proceeds to operation 560.

If, instead, at operation 520 it is determined that a motion event has been detected then, according to another operation 1275, a prompt may be output by an output device of the WCD system. The prompt can instruct to the patient actuate the cancel switch.

At operation 1273, it is determined whether the cancel switch has been actuated, within a preset time after the prompt has been outputted. If not, the operation 560 takes place. If yes, then according to another operation 1238 an aspect of the motion input is caused to be stored in a memory such as memory 238, and operation 560 can be bypassed.

The stored aspect can be used as learning, and the WCD system could remember the profile of an activity during which the cancel button was pressed. For example, it could be then determined by another motion detection input from the motion detector that another motion event was detected. The other, newer motion detection input could be determined to be similar enough with the stored aspect, for example if it meets a similarity criterion. In such embodiments, even if another rhythm analysis is performed, and even if it is determined that the shock criterion is met from that other rhythm analysis, the discharge circuit might not be caused to discharge the electrical charge though the patient responsive to the shock criterion being met from the other rhythm analysis, if the other motion detection input meets a similarity criterion with respect to the stored aspect. In such embodiments, the WCD system might not start a charge sequence and might not alert the patient during a motion profile that had been seen before, and had been determined not to be associated with a shockable rhythm.

In the methods described above, each operation can be performed as an affirmative step of doing, or causing to happen, what is written that can take place. Such doing or causing to happen can be by the whole system or device, or just one or more components of it. In addition, the order of operations is not constrained to what is shown, and different orders may be possible according to different embodiments. Moreover, in certain embodiments, new operations may be added, or individual operations may be modified or deleted. The added operations can be, for example, from what is mentioned while primarily describing a different system, apparatus, device or method. In general, all steps of different methods and features of different embodiments can be combined or exchanged according to the knowledge of a person skilled in the art.

## Claims

1. A wearable cardiac defibrillator (WCD) system, comprising:
a support structure (170) configured to be worn by a patient (82);
an energy storage module configured to be coupled to the support structure and to store an electrical charge;
a discharge circuit coupled to the energy storage module;
a measurement circuit (220) configured to render a physiological input from a patient physiological signal; and
a processor configured to:
determine whether or not a motion detector detected a motion event (382) by a motion detection input from the motion detector or absence of such a motion detection input,
perform a rhythm analysis based on the physiological input in a first manner if it is determined that no motion event (382) has been detected,
perform a rhythm analysis based on the physiological input in a second manner different from the first manner, if it is determined that a motion event (382) has been detected,
determine whether a shock criterion (671, 672) is met from the rhythm analysis that has been performed, and
cause the discharge circuit to discharge the electrical charge through the patient if the shock criterion (671, 672) is met.

2. The WCD system of claim 1, in which
the physiological input is derived from an ECG signal of the patient,
in the first manner the ECG signal is high-pass filtered at a first cutoff frequency, while
in the second manner the ECG signal is high-pass filtered at a second cutoff frequency different from the first cutoff frequency.

3. The WCD system of any of the preceding claims, in which
a motion event (382) has been detected,
the motion event (382) becomes classified as one of walking/running and other,
the rhythm analysis is performed in the second manner if the motion event (382) has become classified as walking/running, and
the rhythm analysis is performed in a third manner if the motion event (382) has become classified as other, the third manner different from the second manner.

4. The WCD system of any of the preceding claims, in which
the motion detector includes an accelerometer configured to output a motion detection signal, and
if it is determined that a motion event (382) has been detected, the processor is further configured to:
classify the motion event (382) as one of ambient and conscious-patient generated according to the motion detection signal, and
if the motion event (382) has become classified as conscious-patient generated, not cause the discharge circuit to discharge.

5. The WCD system of claim 4, in which the processor includes an Analog-to-Digital Converter (ADC) that is used in the classifying.

6. The WCD system of any of the preceding claims, in which
the measurement circuit (220) includes an impedance detector configured to generate an impedance sense input, and
if it is determined that a motion event (382) has been detected, the processor is further configured to:
classify the motion event (382) as one of ambient and conscious-patient generated according to the impedance sense input, and
if the motion event (382) has become classified as conscious-patient generated, not cause the discharge circuit to discharge.

7. The WCD system of any of the preceding claims, further comprising:
a GPS location sensor configured to generate a location sense input, and
if it is determined that a motion event (382) has been detected, the processor is further configured to:
classify the motion event (382) as one of ambient and conscious-patient generated according to the location sense input, and
if the motion event (382) has become classified as conscious-patient generated, not cause the discharge circuit to discharge.

8. The WCD system of any of the preceding claims, further comprising:
a cellphone signal sensor configured to detect an ambient cellphone signal, and
if it is determined that a motion event (382) has been detected, the processor is further configured to:
classify the motion event (382) as one of ambient and conscious-patient generated according to the ambient cellphone signal, and
if the motion event (382) has become classified as conscious-patient generated, not cause the discharge circuit to discharge.

9. The WCD system of any of the preceding claims, further comprising:
a WiFi signal sensor configured to detect an ambient WiFi signal, and
if it is determined that a motion event (382) has been detected, the processor is further configured to:
classify the motion event (382) as one of ambient and conscious-patient generated according to the ambient WiFi signal, and
if the motion event (382) has become classified as conscious-patient generated, not cause the discharge circuit to discharge.

10. A non-transitory computer-readable storage medium storing one or more programs which, when executed by at least one processor of a wearable cardiac defibrillator (WCD) system, in particular according to one of the preceding claims, the system including a support structure configured to be worn by a patient, an energy storage module configured to be coupled to the support structure and to store an electrical charge, a discharge circuit coupled to the energy storage module, and a measurement circuit configured to render a physiological input from a patient physiological signal, the WCD system cooperating with a motion detector, results in:
receiving the physiological input;
determining whether or not a motion detector detected a motion event by a motion detection input from the motion detector or an absence of such a motion detection input;
if it is determined that no motion event has been detected, determining from the physiological input whether a still shock criterion is met and, if so, causing the discharge circuit to discharge the electrical charge through the patient; else
if it is determined that a motion event has been detected, determining from the physiological input whether a moving shock criterion is met and, if so, causing the discharge circuit to discharge the electrical charge through the patient, the moving shock criterion different from the still shock criterion.

11. The medium of claim 10, in which
the determination of whether the still shock criterion is met is made by performing a rhythm analysis based on the physiological input in a first manner, and
the determination of whether the moving shock criterion is met is made by performing a rhythm analysis based on the physiological input in a second manner different from the first manner.

12. The medium of claim 10 or claim 11, in which
the physiological input is derived from an ECG signal of the patient (82),
in the first manner the ECG signal is high-pass filtered at a first cutoff frequency, while
in the second manner the ECG signal is high-pass filtered at a second cutoff frequency different from the first cutoff frequency.

13. The medium of any of claims 10 to 12, in which
a rhythm analysis is performed from the physiological input,
a shock advice parameter is generated from the rhythm analysis,
the still shock criterion is that the shock advice parameter has a value greater than a first threshold, and
the moving shock criterion is that the shock advice parameter has a value greater than a second threshold different from the first threshold.

14. A method for a wearable cardiac defibrillator (WCD) system, in particular according to one of claims 1 to 8, the system including a support structure (170) configured to be worn by a patient (82), an energy storage module configured to be coupled to the support structure and to store an electrical charge, a discharge circuit coupled to the energy storage module, a measurement circuit configured to render a physiological input from a patient physiological signal, the WCD system cooperating with a motion detector that is configured to detect a motion event (382), a value becoming assigned to a motion level parameter in response to the motion detector detecting or not detecting a motion event (382), the method comprising:
receiving the physiological input;
performing a rhythm analysis based on the physiological input and on the assigned value of the motion level parameter;
determining whether a shock criterion (671, 672) is met from the rhythm analysis; and
discharging the electrical charge through the patient (82) if the shock criterion (671, 672) is met.

15. The method of claim 14, further comprising:
assigning the value to the motion level parameter.
